# EUROPEAN PATENT APPLICATION

(11) **EP 2 787 347 A1**
(43) Date of publication of application: **08.10.2014**
(21) Application number: 13162106.2
(22) Date of filing: 03.04.2013
(51) Int. Cl.: G01N 33/564

(54) **Method for detecting Aß-specific antibodies in a biological sample**

(71) Applicant: Affiris AG, 1030 Wien (AT)
(72) Inventor: Staffler, Günther, 1030 Vienna (AT); Mairhofer, Andreas, 1030 Vienna (AT); Schmidt, Walter, 1020 Vienna (AT)
(74) Representative: Sonn & Partner Patentanwälte

(57) **Abstract**

Disclosed is a method for detecting Aβ-specific antibodies in a biological sample comprising the following steps:
- contacting the sample with Aβ-variant aggregates or with particles comprising Aβ-variant aggregate like surfaces and allowing the Aβ-specific antibodies to bind to the Aβ-variant aggregates, and
- detecting the Aβ-specific antibodies bound to the Aβ-variant aggregates by a single particle detection technique, preferably by fluorescence activated cell sorting FACS.

## Description

The present invention relates to methods for detecting Aβ-specific antibodies in biological samples, especially in connection with and for diagnosing of Alzheimer's disease (AD).

AD is a complex progressive disorder that involves interacting pathological cascades, including amyloid-β aggregation and plaque formation in the brain, hyperphosphorylation of tau protein with formation of intraneuronal tangles. Concomitant to the aggregation and the hyperphosphorylation of these cerebral proteins inflammatory processes contribute to the loss of the synaptic integrity and progressive neurodegeneration.

The conversion of amyloid β peptide (Aβ or A-beta) from a soluble form with mainly alpha-helical or random coil secondary structure to an aggregated form with cross-beta-sheet secondary structure, that finally forms fibrils and amyloid plaques in the brain, represents one of the first hallmarks of AD pathology. Several forms of Aβ, C- as well as N-terminally truncated or modified peptides, contribute to Aβ plaque formation in the brain. The three major C-terminal variants of Aβ include the peptides Aβ1-40 (consisting of 40 amino acids (aa) with Val-40 as last aa), Aβ1-42, and Aβ1-43. Beside these major forms of C-terminal truncated peptides also other truncated forms exist that appear less frequently, namely Aβ1-37, Aβ1-38, and Aβ1-39. The N-terminal variants of Aβ consist of Aβ3-40/42/43 and Aβ11-40/42/43. In all these N-terminal truncated forms the glutamic acid holds the first position. This aa is not stable but rather undergoes a change to build the pyroglutamate (pE) resulting in the formation of Aβp(E)3-40/42 and Aβp(E)11-40/42.

Positional scanning experiments of the Aβ peptide by replacing each single amino acid by a neutral amino acid (either alanine or proline) identified one region within the Aβ peptide which is important for the fibril and aggregation formation process. Point mutations within Aβ have identified residues 16-20 (KLVFF) as core sequence for fibril formation (Moss et al., Mol Pharmacol 64 (2003): 1160-1168). In other experiments where a library of trimeric to decameric peptides spanning the entire Aβ1-40 sequence was synthesized and screened for their ability to bind the full-length peptide, again the KLVFF penta-peptide was found to be the minimum sequence required to bind Aβ1-40, again indicative that this penta peptide constitutes the amyloidogenic core sequence and that this five amino acid long stretch is enough to induce or support fibril formation. Furthermore, alanine substitution of this core sequence subsequently showed that Lys16, Leu17, and Phe20 are critical for this interaction (Moss et al., 2003). The stereospecific binding of KLVFF to the homologous sequence in Aβ was later confirmed and shown to result from specific hydrophobic and electrostatic interactions (Moss et al., 2003).

Alanine replacement of residues 15 and 21-36, residues adjacent to the KLVFF core sequence, leads to a destabilization of the fibril formation process (Williams et al., J. Mol. Biol. 335 (2004): 833-842)). In addition, it has been shown that positions 15-21, 24-28, and 31-36 within amyloid structure are sensitive and positions 18-21, 25-26, and 32-33 within amyloid structure are particularly sensitive to the main-chain disrupting effects of proline replacements (Williams et al., J. Mol. Biol. 357 (2006): 1283-1294). This indicates that beside the core sequence KLVFF an additional moiety in the Aβ peptide, which is located C-terminally to the core sequence is involved in fibril formation. In line with these data, are results coming from other studies showing that a C-terminal fragment of Aβ1-40, namely Aβ25-35 exhibits amyloid fibril formation by itself. While the N-terminal region of Aβ25-35 is hydrophilic, the C-terminal region is highly hydrophobic, which is assumed to form the central core stabilizing the Aβ amyloid fibril. Because of this similarity, Aβ25-35 is often used as a model of Aβ1-40 for studying fibril formation and cytotoxicity (Ban et al., 2004).

According to a recently proposed solid-NMR-based structure of Aβ, residues 12-24 and 30-40 adopt β-strand conformations combined by hydrophobic interactions, while residues 25-29 assume a bend that brings the two β-strands in contact via hydrophobic side chain interactions (Ban et al., J. Mol. Biol. 344 (2004): 757-767). On the other hand, as mentioned above mapping Aβ amyloid fibril secondary structure using scanning proline mutagenesis suggested that residues 15-21, 24-28, and 31-36 adopt β-strand conformations.

Studies using cystein substitution mutants in Aβ1-40 to probe amyloid fibril structure and stabilization identified again residues 16-19 and 31-34 as core sequences for amyloid fibril formation. In contrast to alanin and prolin mutation studies which identified residue 21 as being important for Aβ fibril formation, cysteine substitution on position 21 did not interfere with this aggregation process (Shivaprasad et al., JBC Vol. 281 No. 2 (2006): 993-1000). Furthermore, in this study multiple amino acid exchanges in Aβ1-40 have been performed showing that more than one amino acid (that do not constitute the core sequences) can be exchanged without major interference with Aβ fibril or aggregate formation.

Importantly, residues 1-14 and 37-40/42 are relatively insensitive to amino acid replacements and thus are likely to exist in relatively unstructured, flexible elements extruding from the β-sheet-rich amyloid core. A second group of amino acids which is relatively insensitive to amino acid replacements contains the residues 22, 23, 29, and 30. These residues are likely to occupy turn positions between sequences forming the cross-β-sheet structure in Aβ fibrils (Williams et al., 2004). Thus all these amino acids seem to play a minor role in amyloid fibril formation.

Until recently, the diagnosis of AD was a purely clinical one based on the gradual occurrence of cognitive deficits in at least two domains (e.g., cognition, function), negatively affecting the patient's everyday life without another discernable cause (e.g., vascular). The limitation of clinical diagnosis of AD are high rates of misdiagnoses (diagnostic specificity of 80% by experts) and the fact that the diagnosis could only be made at a late time point when the disease had caused substantial neuronal loss that resulted in functional deficits.

Based on knowledge gathered through the last two decades, the way of diagnosing AD is rapidly changing. A group of researchers led by B. Dubois, Paris, are the first to integrate data, which had emerged from investigations into the pathology underlying AD, into the diagnostic algorithm (Dubois et al., Lancet Neurol.9 (2010): 1118-1127). According to the authors, the diagnosis of AD is based on a specific cognitive deficit (an alteration of the episodic memory) which has to occur in combination with a change in disease-specific biomarkers (hippocampal atrophy as assessed by structural MRI; AD-typical cerebrospinal fluid signature (low A42, high total tau, high phosphoTau); positive amyloid imaging; defined genetic risk). Recently, this pathophysiology driven diagnostic algorithm has been largely adopted by the NIH-NINCDS working group (McKhann et al., Alzheimer's & Dementia 7 (2011): 263-269). For mainly practical purposes, the NIH NINCDS working group kept the diagnosis of MCI (mild cognitive impairment) of the AD-type as an early stage of AD.

The concept of enhancing the clinical diagnosis of AD by means of biomarker reflecting the pathology of the disease has been adopted by a working group installed by the National Institute of Aging (NIA, NIH, USA) and the Alzheimer Association. By doing so, AD is not any more a diagnosis by exclusion but begins to be a positive one. The fact that NIA and AA did not completely follow the biomarker-driven algorithm proposed by Dubois and colleagues reflects the limitations of the currently available biomarkers. The AD cerebrospinal fluid (CSF) signature may be discussed as an example. The CSF of AD patients shows a typical pattern, namely reduction of Aβ1-42 and elevation of total Tau (tTau) and phosphoTau (pTau). The signature is present in AD patients but does not detect a change over time. In fact, in a population of patients at risk for AD (i.e., MCI patients) it identifies the ones who move on to develop the clinical symptoms. Already at that stage, the CSF shows the same expression pattern and amount of change as in patients with full-blown AD. Thus, while it is not possible to define a normal range for Aβ1-42, tTau and pTau, there is no definition of turning points, i.e. the moment in a given patient when for example the Aβ physiology switches from normal to pathological. The very same is true for any of the currently followed and not yet validated biomarkers. The main reason for this is that there are only a few longitudinal studies assessing this issue because it is not easily possible to repeat CSF-, MRI-, amyloid-imaging examinations because of the risk they impose onto patients and/or the costs associated with them.

Thus, there is still a lack of a reliable biomarker that can be applied repetitively, at low risk and costs. This is especially true since all efforts taken so far to develop blood-based AD biomarkers failed (Hampel et al., Nat. Rev. Drug Discov. 9 (2010): 560-574). The availability of such a biomarker would be of outmost importance for the development of disease-modifying therapies. The earlier such therapies would be administered, the bigger the chances for success. And, one could limit such efforts to true AD cases identified with the help of a specific biomarker.

Thus far, Aβ (various Aβ species and aggregation states tested) have been evaluated in AD and MCI, the pre-dementia stage of AD. Recent findings show that there is an anti-amyloidogenic-activity of IgG and IgM contained in plasma and cerebrospinal fluid of AD patients and healthy controls (O'Nuallain et al., J. Clin. Immunol.30 (2010) Suppl.1: S37-S42; Marcello et al., J Neural. Transm.116 (2009): 913-920). Results obtained by ELISA or immunoprecipitation assays assessing IgG and/or IgM specific for various Aβ forms/aggregation states showed that AD- and MCI patients exhibit lower levels of serum Aβ auto-antibodies than healthy controls. Although these studies showed a difference in auto-antibody concentration, the used methods lack the sensitivity and specificity that would be necessary to use them as predictive diagnostic tool to identify AD- or MCI patients with high selectivity and specificity. Most of the methods used so far are based on ELISA technology. To increase the sensitivity in these assays some approaches use radioactive labelling of the Aβ1-42 peptide. The ROC (receiver operating characteristic) analysis of Brettschneider et al., (Brettschneider et al., Biol. Psychiatry 57 (2005): 813-817), reached a specificity of 46.7% when sensitivity was set at 81.3% using an immunoprecipitation assay with chloramine T labelled Aβ1-42 to measure the difference between healthy controls and AD patients. In contrast Bayer et al. (WO 2010/128139 A1; Marcello et al., J Neural. Transm.116 (2009): 913-920) used an ELISA based method where a pyroglutamate-Aβ fragment was coated on the plate. In this case, the detection of anti-Aβ specific IgM-autoantibodies in healthy controls and AD patients with an anti-IgM-HRP antibody showed that specificity was 60% when sensitivity was set to 80%. So far, none of these methods fulfilled the criteria that would qualify them as predictive biomarker (>80% specificity) for AD.

Not known is the reason for the reduced serum concentration of Aβ-specific antibodies in these two groups of patients. There are two general and non-mutually exclusive explanations: reduced production (disease-specific versus general immunosenescence) and redistribution (antibodies are trapped in amyloid deposits present for example in the brain). Support for a potential function of these Aβ-specific (auto)antibodies comes from recent studies demonstrating that commercially available blood products, namely the pooled IgG fraction extracted from plasma derived from healthy donors, has been shown to contain antibodies specific for Aβ peptides. Two such products, intravenous immunoglobulin (IVIG) preparations of two different companies, are currently undergoing clinical testing to evaluate their potential to interfere with or prevent AD pathology.

Another unanswered question is whether the level of Aβ-specific antibodies is reduced in disease entities with an Aβ component in their pathophysiology, namely Parkinson's dementia (PDD), Dementia with Lewy Bodies (DLB), Cerebral Amyloid Angiopathy (CAA), chronic head trauma (e.g., boxing). Investigation of the level of Aβ-aggregate-specific antibodies in these diseases could add to the present understanding of the processes that result in the reduction of the serum concentration of Aβ-aggregate-specific antibodies in AD. Investigation of sera of patients with these diseases could clarify the question as to whether AD results from a specific immunodeficiency (in the case where Aβ-specific antibody titers are only reduced in AD but not in other diseases characterized by Aβ pathology) or whether the reduced levels of Aβ-specific antibodies result from redistribution due to extensive Aβ pathology. In the former case, the test would be a highly disease-specific biomarker and, as a result, should be helpful in differentiating AD from the aforementioned disease entities. Assuming the second scenario, the test could qualify for the identification of patients whose disease is driven by Aβ pathology.

In WO 2010/128139 A1, biomarkers and methods for diagnosing AD are disclosed, especially antibodies against pGlu Aβ. Funke et al. (Curr. Alz. Res., 6 (3) (2009): 285-289) discussed whether the detection of Aβ aggregates in body fluids is a suitable method for early diagnosis of AD. Maetzler et al. (J. Alz. Dis. 26 (1) (2011): 171-179) reported that autoantibodies against amyloid and glial-derived antigens are increased in serum and cerebrospinal fluid of Lewy body-associated dementias. Funke et al. (Rejuv. Res. 13 (2-3) (2010): 206-209) disclosed a single-particle detection system for Aβ aggregates. Fukumoto et al. (Faseb J., 1 (24) (2010): 2716-2726) disclosed that high-molecular weight β-amyloid oligomers are elevated in cerebrospinal fluid of AD patients. Methods for the detection of Aβ-specific antibodies by Aβ-aggregates are disclosed in PCT/EP2012/068494.

Although AD is considered to be a degenerative brain disease, also the immune system has an important role in the disease process. In the recent years, immune-based therapies are designed to remove amyloid-beta peptide from the brain. These therapy concepts gave positive results in animal models of the disease. Clinical trials of active amyloid-beta vaccination of patients with Alzheimer's disease were discontinued after some patients developed Meningoencephalitis. New immunotherapies using humoral and cell-based approaches are currently being investigated for the treatment and prevention of Alzheimer's disease and also other related diseases. In such immunotherapeutic approaches, there is an unmet need for reliable biomarkers which monitor or evidence immune responses of the patients during treatment and development of the disease. There is also a need to identify relevant Aβ antibodies indicative of the vaccination aim in the vaccination patients.

It is an object of the present invention to provide means for improving the diagnosis of AD, especially for tracking early stages of the disease and for observing the development of clinical trials for drugs for the treatment of AD. It is a further object to provide reliable tools for detecting anti-Aβ antibodies in biological samples, especially in samples of human AD patients or human individuals who are suspected to have or are at risk of developing AD.

Therefore, the present invention provides a method for detecting Aβ-specific antibodies in a biological sample comprising the following steps:
- contacting the sample with Aβ-variant aggregates or with particles having Aβ-variant aggregate like surfaces and allowing the Aβ-specific antibodies to bind to the Aβ-variant aggregates, and
- detecting the Aβ-specific antibodies bound to the Aβ-variant aggregates by a single particle detection technique, preferably by fluorescence activated cell sorting (FACS),
wherein the Aβ-variant is selected from the group consisting of
- a mutant of the Aβ-1-40- or the Aβ-1-42-peptide containing at least one mutation at an amino acid position other than amino acids 16 to 20, 25 to 28 and 31 to 35,
- a naturally occurring Aβ-peptide other than Aβ-1-40, Aβ-1-42, Aβ-1-43, Aβ-3-42 or Aβ-p(E)3-42,
- a truncated form of the Aβ-1-40- or the Aβ-1-42-peptide, wherein the truncation is at amino acid positions other than amino acids 16 to 35 and wherein the truncation is at least one amino acid and at most 20 amino acids; or
- combinations thereof, especially combinations of at least one mutation and at least one truncation of the Aβ-1-40- or the Aβ-1-42-peptide.

With the present invention, a new method to detect Aβ-specific auto-antibodies is disclosed which can be used as diagnostic tool which is extremely helpful in diagnosing AD and monitoring the development of AD. The method according to the present invention has turned out to be specifically suited for monitoring the development of clinical trials using an immunotherapeutic approach to treat AD. The present method is based on the invention that not just single Aβ peptides are used as capturing tools for the Aβ-specific antibodies, but that instead Aβ-variant aggregates are used and that thereby generated antibody-Aβ-variant aggregate complexes are detected using a single particle detection technique.

The Aβ-variants used according to the present invention are selected from the group consisting of
- a mutant of the Aβ-1-40- or the Aβ-1-42-peptide containing at least one mutation at an amino acid position other than amino acids 16 to 20, 25 to 28 and 31 to 35,
- a naturally occurring Aβ-peptide other than Aβ-1-40, Aβ-1-42, Aβ-1-43, Aβ-3-42 or Aβ-p(E)3-42,
- a truncated form of the Aβ-1-40- or the Aβ-1-42-peptide, wherein the truncation is at amino acid positions other than amino acids 16 to 35 and wherein the truncation is at least one amino acid and at most 20 amino acids; or
- combinations thereof, especially combinations of at least one mutation and at least one truncation of the Aβ-1-40- or the Aβ-1-42-peptide.

Preferred peptide variants according to the present invention include truncations and/or modifications of Aβ1-40/42 (i.e. peptides containing amino acid exchanges; the number of exchanges may be more than 10, however, preferred variants have 10 to 1 exchanges, more preferred 5 to 1 exchanges, even more preferred 4, 3, especially a single exchange.

The numeration of amino acids is based on the established numeration of Aβ-peptides:
Aβ-1-42 (SEQ ID NO.1): 

1               5                   10                  15

            20                  25                  30

        35                  40
Aβ-1-40 (SEQ ID NO.2): 
Asp Ala Glu Phe Arg His Asp Ser Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val Gly Gly Val Val
        35                  40
Aβ-1-43 (SEQ ID NO.3): 
Asp Ala Glu Phe Arg His Asp Ser Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val Gly Gly Val Val Ile Ala Thr
        35                  40
Aβ-3-42 (SEQ ID NO.4): 
Glu Phe Arg His Asp Ser Gly Tyr Glu Val His His Gln Lys Leu Val
1               5                   10                  15
Phe Phe Ala Glu Asp Val Gly Ser Asn Lys Gly Ala Ile Ile Gly Leu
            20                  25                  30
Met Val Gly Gly Val Val Ile Ala
        35                  40
Aβ-p(E)3-42 (SEQ ID NO.5):,

pGlu Phe Arg His Asp Ser Gly Tyr Glu Val His His Gln Lys Leu Val
1               5                   10                  15
Phe Phe Ala Glu Asp Val Gly Ser Asn Lys Gly Ala Ile Ile Gly Leu
            20                  25                  30
Met Val Gly Gly Val Val Ile Ala
        35                  40

The following groups of Aβ variants are specifically preferred in Aβ-variant aggregates (group 4 being the most preferred, followed by group 3, then group 2 and group 1)

Group 1 of Aβ variants: peptides containing either the core sequence Aβ16-20 or the second core sequence Aβ25-35. These peptides have been shown to form amyloid fibrils by themselves. Starting from these core sequences peptides can be extended by amino acids present in Aβ peptide or by alternative amino acids forming either truncated native Aβ peptides or truncated-modified Aβ peptides.

Group 2 of Aβ variants: peptides containing the sequence Aβ16-20-X₂₁₋₂₃-24-28-X₂₉₋₃₀-31-36 (meaning that aa 16 to 20, 24 to 28 and 31 to 36 are present and linked by a 3 and a 2 aa linker (of arbitrary primary sequence)). Starting from this sequence peptide can be extended by amino acids present in Aβ peptide or by alternative amino acids forming either truncated native Aβ peptides or truncated-modified Aβ peptides.

Group 3 of Aβ variants: peptides containing the sequence Aβ16-36. Starting from this sequence peptide can be extended by amino acids present in Aβ peptide or by alternative amino acids forming either truncated native Aβ peptides or truncated-modified Aβ peptides.

Group 4 of Aβ variants: peptides containing the sequence Aβ11-36 or Aβp(E)11-36. Starting from this sequence peptide can be extended by amino acids present in Aβ peptide or by alternative amino acids forming either truncated native Aβ peptides or truncated-modified Aβ peptides.

Mutations are preferably conservative mutations, i.e. amino acid mutations of residues having similar side chains. A conservative mutation substitutes an amino acid in the polypeptide with an amino acid within the same or similar defined class of amino acids. For example, A, V, L, or I can be conservatively mutated to either another aliphatic residue or to another nonpolar residue. Conservative amino acid substitutions can frequently be made in a polypeptide without altering either the conformation or the function of the polypeptide. Substitutions can be chosen based on their potential effect on (a) backbone structure in the vicinity of the substitution, for example, a sheet or helical conformation, (b) the charge or hydrophobicity of the molecule at the target site, or (c) the volume and branching of the side chain. In general, amino acid residues can be classified based on side-chain properties: (1) aliphatic: A, M, V, L, I; (2) small aliphatic: A, V; (3) large aliphatic: M, L, I; (4) neutral hydrophilic: S, T, N, Q; (5) acidic: D, E; (6) basic: H, K, R; (7) charged: R, D, E, H, K; (8) residues that affect backbone conformation: G, P; and (9) aromatic: W, Y, F. Non-conservative mutations substitute a member of one of these classes for a member of a different class. Conservative mutations according to the present invention substitute a member of one of these classes for another member of the same class (except class (8)). Generally mutations are not made to C, G and P.

Preferred aa positions that are mutated according to the present invention are between 11 to 16 and 36 to C-terminus (e.g. 40, 42). Here, also non-conservative exchanges (preferably to small neutral or aliphatic amino acids) can be performed. In the loop region it is preferred to only provide (conservative) mutations at amino acid residues 21 to 23 and 29 to 30. In the N-terminal region (e.g. residues 1-10), virtually all mutations and truncations are possible (conservative and non-conservative), aggregate formation according to the present invention is usually not affected by this region.

Numerous variants according to the present invention are specifically disclosed herein; however other variants, as defined above can be applied in the course of the present invention, provided that they still have the ability to form aggregates. It follows that any mutated/truncated variant (or combinations thereof; within the teaching as disclosed herein) can be easily tested with respect to its aggregate-forming capabilities by testing the variant whether it allows aggregate formation in the manner described herein:

Briefly, Aβ-variant aggregates (derived from different Aβ truncated and modified versions) are generated e.g. by overnight incubation, specifically as disclosed in the example section. Subsequently, Aβ-variant aggregates are incubated with serum samples derived either from healthy donors (HD) or from AD patients to allow binding of present antibodies (both IgG and IgM). Antibodies bound to Aβ-variant aggregates can be detected by any suitable method available to a person skilled in the art, e.g. by using a labelled secondary antibody which recognises the Aβ-specific antibody bound to the Aβ-variant aggregates. For example a phycoerythrin (PE)-labelled secondary antibody can be used. Thereafter, the immune complexes comprising the Aβ-specific antibody bound to the Aβ-variant aggregates (and optionally one or more detection agents, such as secondary antibodies) are measured using a single particle detection technique, such as FACS (fluorescence activated cell sorting) - analysis also known as flow cytometry. Using the method according to the present invention, it can be shown that AD patients contain less Aβ-specific immunoglobulins which are reactive towards the Aβ-variant aggregates (free Aβ-specific immunoglobulins) provided according to the present invention compared to healthy subjects. Furthermore, using the method according to the present invention, it can be shown that reactivity of Aβ-specific immunoglobulins (towards the Aβ-variant aggregates provided according to the present invention) derived from AD patients can be increased by a procedure known as demasking (removing of potentially bound Aβ-antigens from autoantibodies). This is in contrast to the reactivity of Aβ-specific immunoglobulins from healthy subjects where such an increase of reactivity towards the Aβ-variant aggregates cannot be detected after treating these sera in a special way. On the other hand, the reactivity of IgM-antibodies after demasking (=dissociation of already bound Aβ in the serum) reveal an increased level of IgM in AD patients. Additionally, also the difference between IgG levels with and without demasking are determined (delta (Δ) values). This parameter is also elevated in AD patients as compared to healthy controls showing higher antibody occupancy by Aβ of antibodies in the pathological state of the disease. Furthermore, with the present invention data are provided showing that the present method has a much higher capacity to detect Aβ-specific antibodies and thus has a much higher power to diagnose AD compared to methods published so far. Given these facts, the method according to the present invention fulfils the theoretical prerequisites of a predictive diagnostic tool to identify AD and to follow the clinical response of a given patient to treatment.

Aggregates according to the present invention can also be composed of more than one species, i.e. the Aβ-variants according to the present invention can be combined with each other or with naturally occurring Aβ-peptides, especially Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42. According to a preferred embodiment of the present invention, the aggregate according to the present invention comprises at least two different variants according to the present invention or at least one variant according to the present invention combined with at least one of Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42. Other embodiments comprise at least three variants according to the present invention and/or at least two different Aβ-peptides selected from Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, especially Aβ1-40, Aβ1-42, Aβ3-42 and Aβp(E)3-40/42. Molar ratios of the variants in the aggregates can be easily adapted and optimised for specific mixed aggregates. Preferably the molar ratio in a mixture of 2 different species is between 1:100 and 100:1, more preferred 1:10 to 10:1, especially 1:5 to 1:5; preferred mixtures with more than two different species can be provided along this general rule. According to a separate aspect, the present invention also provides mixed aggregates that are composed of at least two of Aβ1-40, Aβ1-42, Aβ1-43, Aβ1-37, Aβ1-38, Aβ1-39, Aβ3-40/42/43, Aβ11-40/42/43, Aβp(E)3-40/42 and Aβp(E)11-40/42, preferably a mixed aggregate comprising Aβ1-40, Aβ1-42, Aβ3-42 and Aβp(E)3-40/42, especially a mixed aggregate comprising Aβ1-42.

Another preferred embodiment of the Aβ-variant-comprising-aggregates according to the present invention relates to aggregates that comprise - besides the at least one Aβ-variant as disclosed herein - an aggregate component of a different aggregate forming polypeptide species (as used herein, the term "polypeptide" is covering the term "protein" and usually these two terms are used synonymously; the term "protein" is therefore applied herein also for polypeptides with less than 100 or less than 50 amino acid residues). Preferably, this further aggregate-forming polypeptide species is selected from the group consisting of Tau protein or an aggregate-forming variant thereof, alpha synuclein (aSyn) or an aggregate-forming variant thereof; islet amyloid polypeptide (IAPP) and an aggregate-forming variant thereof, especially IAPP-Npro, TAR-DNA binding protein 43 (TDP43) or an aggregate-forming variant thereof; and Superoxide-Dismutase 1 (SOD1) or an aggregate-forming variant thereof.

Preferred aggregate forming variants of aSyn ("aSyn variants") are aSyn-126 (lacking aa 41-54; loss of exon 4), aSyn-112 (lacking aa 103-130; loss of exon 5), aSyn-98 (lacking exon 3 and 5), phosphorylated aSyn, aSyn-126, aSyn-98 and aSyn-112, especially Ser129 phosphorylated aSyn, nitrosylated/nitrated, acetylated and mono-, di-, or tri-ubiquitinated aSyn, aSyn-126 and aSyn-112, or C-terminally truncated forms of aSyn, aSyn-126 and aSyn-112, preferably aSyn lacking the C-terminal 20, 21, 22, 23, 24, 25, 26, 27, 28, 29 or 30 amino acids.

Preferred aggregate forming variants of Tau ("Tau variants") are hyperphosphorylated Tau, abnormally phosphorylated Tau (as referred to and defined in Shahani et al. J. Neurosci. 26 (2006), 6103-6114), Tau protein variants that have marker function for a disease (either isoform of all 6 naturally occurring isoforms (Tau441, Tau412, Tau410, Tau383, Tau381, Tau352) or mutant forms thereof (e.g. P301L, P301S, V337M, etc.), e.g.: forms preferably simultaneously phosphorylated at amino acids 181, 202, 205, 212, 214, 231, 396 and, optionally at additional residues present in Tau like 18, 153, 175, 189, 235, 262, 394, 404 and 422, of Tau441, Tau412, Tau410, Tau383, Tau381, Tau352 (a more detailed analysis of Tau phosphorylation is disclosed e.g. in Hanger et al., Trends in Mol. Med. 15 (2009), 112-119)."

The present invention was developed for the analysis of Aβ-specific antibodies in human samples. It is therefore a preferred embodiment to detect human Aβ-specific antibodies, preferably human IgG or IgM antibodies, especially human IgG antibodies. As already mentioned, the detection of Aβ-specific antibodies in humans is known in principle in the art; however, the role as a possible biomarker could not be verified. As shown with the present invention, this was also due to the analytical insufficiency of the detection methods available in the art. Due to the superiority of the method according to the present invention, the availability of these antibodies in human samples as biomarkers is enabled. The present method is therefore specifically suited to detect autoantibodies in biological samples. Accordingly, in a preferred embodiment of the present method, the Aβ-specific antibodies to be detected and quantified are autoantibodies.

In contrast to prior art methods, the present method uses Aβ-variant aggregates as probe for binding the Aβ-specific antibodies from the samples. Although such aggregates are known in principle in the art, it was not realised that the use of such aggregates in the analysis of Aβ-specific antibodies, especially in human samples, could significantly improve such methods, also in combination with the single particles detection techniques, such as FACS. Due to the use of such aggregates, the detection with single particles detection techniques (which are established techniques in various different fields and for different questions) is possible for analysing Aβ-specific antibodies in human samples (such as blood) which are usually very complex and difficult to handle.

Preferably, the dimensions of the aggregates to be used according to the present invention are standardised for analytical use. This can be done by establishing certain parameters during production of the aggregates. Depending on the conditions applied during generation of the aggregates, the size of the aggregates can be adjusted. Preferred sizes of the Aβ-variant aggregates according to the present invention are from 50 nm to 15 µm, preferably from 100 nm to 10 µm, especially from 500 nm to 5 µm (defined by the length of the aggregates (i.e. the longest extension).

A preferred method to provide aggregates suitable for the present invention comprises the step of incubating Aβ-variants according to the present invention, at a pH of 2 to 9 for at least 20 min, preferably at least 1 h, especially at least 4 h. The duration of incubation is one of the parameters to adjust the size of the aggregates: the longer the incubation, the larger are the aggregates. Typical incubation times are from 10 min to 24 h. Shorter incubation times usually result in only very short aggregates and low number of aggregates; the aggregates produced with significantly longer incubation times than 48 h are usually not preferred in the present method. Of course, aggregates may also be sorted and "sieved" to arrive at the desired size, if needed, e.g. by fractionated centrifugation and similar techniques.

According to the present method, the samples wherein the Aβ-specific antibodies are to be detected are contacted with the Aβ-variant aggregates to achieve binding of the Aβ-specific antibodies possibly present (and reactive vis-a-vis Aβ-variant aggregates) in the samples. The concentration of the Aβ-variant aggregates has therefore to be adjusted in order to provide enough binding positions for the antibodies. Accordingly, the concentration of the Aβ-variant aggregates for binding the antibodies in the sample is preferably in the range of 0.001 to 1 µM, preferably 0.01 to 0.1 µM. The optimal concentration is also dependent on the nature of antibodies to be bound, the nature of the sample, the planned contact time and the size of the aggregates.

The present method is mainly aimed for application on human samples. It is therefore preferred that biological sample is human blood or a sample derived from human blood, preferably human serum or human plasma; human cerebrospinal fluid or human lymph. With such sample sources, also serial and routine testing may be established (especially for samples derived from blood).

Preferred contact times which allow proper binding of the antibodies in the sample to the aggregates are at least 10 min (e.g. 10 min to 48 h), preferably from 15 min to 24 h, especially from 30 min to 2 h.

If the biological sample is not specifically pre-treated, the Aβ-specific antibodies which have a binding capacity to the Aβ-variant aggregates will be bound during contact with the Aβ-variant aggregates. The Aβ-specific antibodies which are masked in the sample (i.e. those antibodies which are already bound to a binding partner (e.g. an Aβ-comprising structure, or endogenous Aβ peptides)) will not be detected by the method according to the present invention (absent such specific sample pretreatment). Whereas the identification and quantification of only reactive antibodies will in many cases be sufficient and desired, there may be situations or diagnostical questions where the total amount of Aβ-specific antibodies in the sample should be detected (reactive and unreactive) or all, the number of reactive Aβ-specific antibodies, the unreactive ("masked") and the total number of Aβ-specific antibodies.

Therefore, according to another preferred embodiment of the present invention, the samples are demasked, i.e. the Aβ-specific antibodies are "freed" from any binding to binding partners present in the sample previous to the contact with the Aβ-variant aggregates according to the present invention. This allows detection of all Aβ-specific antibodies in the sample and not only detection of those antibodies which are not bound to a binding partner in the sample ("free" or "reactive" antibodies). In the course of the present invention it was determined that the amount of reactive Aβ-specific antibodies, especially reactive Aβ-specific IgG, was a key marker for the diagnosis and development of AD. The present method is, as stated above, also suitable for determining the overall amount of Aβ-specific antibodies in a sample, i.e. the free (or "reactive") antibodies as well as those antibodies which are already bound (e.g. to Aβ structures) in the sample. This can be helpful in establishing the difference (Δ) of reactive vs. non-reactive antibodies in a sample, a parameter which is also of significant importance for AD diagnosis. Whereas such difference is not present (or low) in persons with "healthy status" concerning AD, this difference is crucial for the marker function in AD, concerning both Aβ-specific IgG and Aβ-specific IgM. In order to use Aβ-specific IgM as parameter for AD diagnosis, a demasking step prior to performance of the present method is in particular preferred, thus the difference (Δ) of reactive vs. non-reactive antibodies in the sample will be defined and used as relevant parameter.

The method according to the present invention applies a single particle detection technique. Such techniques allow identifying and quantifying ("count") the number and amount of "positive" binding results of the Aβ-specific antibody to the Aβ-variant aggregates. A preferred embodiment of this technology is FACS which is an established technique in the present field. Other detection methods to be used to detect the antibodies bound to the Aβ-variant aggregates are e.g. Luminex or mass cytometry.

According to the Luminex technology, sample preparation may be performed as described in Material and Methods. Following sample preparation Aβ-variant aggregates recognized by specific Aβ-specific antibodies may be detected by a secondary antibody coupled to fluorescent-dyed microspheres which can be detected in multiplex detecting systems e.g. a Luminex reader (Binder et al., Lupus15 (2005):412-421).

If mass cytometry is used as single particle detection technique, sample preparation may also be performed as described in Material and Methods of the example section below. Sample preparation is done as described. Following sample preparation Aβ-variant aggregates recognized by specific Abs may be detected by a secondary antibody coupled to stable isotopes of transition elements which can be detection by atomic mass spectrometry. The sample can then be sprayed through an argon plasma filled inductive coil heated to a temperature of >5,500 K. The sample is vaporized and ionized into its atomic constituents, and the number of the isotope-tagged antibody is quantified by time-of-flight mass spectrometry (Janes et al., Nat. Biotechnol. 29 (2011): 602-604).

Alternatively it is also possible to apply single particle detection techniques where one binding partner (Aβ-variant aggregates or antibody/serum) are immobilized but binding is measured under flow conditions. Examples are the Hybcelltechnology and the Surface Plasmon Resonance technology. Using the Hybcell technology in the present invention, serum samples can be spotted on the surface of the Hybcell (a rotating cylinder) and incubation can be performed with directly fluorescence-labelled preincubated Aβ-variant aggregates or alternatively with a fluorescence-labelled monoclonal Aβ-specific second antibody. Antibodies bound to Aβ-variant aggregates are detected with a laser (Ronacher, Anagnostics Technical Note ANA-TN-005 (2010)). If Surface Plasmon Resonance is used in the method according to the present invention, a reverse setup can be applied: the preincubated Aβ-variant aggregates can be immobilized on a chip surface. The binding of Aβ-specific antibodies from serum to the Aβ-variant aggregates on the chip can be detected by increase of mass on the chip surface and therefore no labelling of the binding partners is necessary. To increase sensitivity or determine IgG-subtypes a serial injection of anti-IgG-AB is possible (Cannon et al., Anal. Biochem. 328 (2004): 67-75). Instead of directly immobilizing Aβ-variant aggregates to the chip surface a capture antibody can be used. For this setup an Aβ-specific antibody is immobilized on the chip surface followed by the injection of preincubated Aβ-variant aggregates. After the capturing of the aggregates serum is injected and reactivity is measured by increase of mass.

Detection of the binding of Aβ-specific antibodies to the Aβ-variant aggregates according to the present invention can be performed by any suitable method known e.g. Fluorescence Spectroscopy (Missailidis et al., Methods in Molecular Biology 248 (2003): 431-441) for detecting the Aβ-specific antibodies bound to the Aβ-variant aggregates by a secondary antibody (e.g. a secondary labelled anti-IgG- or anti-IgM-antibody).

Detection of autoantibodies bound to aggregates can also be performed using substrates specifically binding antibodies such as Protein A or Protein G. Another possibility is to precipitate Aβ-variant aggregate specific autoantibodies using the Aβ-variant aggregates, wash the complex and biotinylate the antibodies. Subsequently streptavidin can then be used as second step reagent.

According to a preferred embodiment of the present invention, the particles having Aβ-variant aggregate like surfaces are beads with Aβ-variant aggregates immobilised on their surface, especially magnetic beads.

The present method is specifically suited for using in connection with AD diagnosis. With the present invention, Aβ-specific autoantibodies in human patients are provided as markers for AD status. People with "normal" level of Aβ-specific antibodies in their blood are "healthy" with respect to AD. If this level is modified in a patient with AD or subjects with a risk of developing AD or being suspected to have AD, such modification level correlates with AD. A "modified" level may be a modification of the absolute number of the Aβ-specific antibodies or a modification of the reactivity of the totality of the Aβ-specific antibodies (e.g. of a given class of Aβ-specific antibodies (IgG, IgM, etc.). For example, decreased reactive Aβ-specific IgG correlates with and is a marker for AD. On the other hand, with IgM, changes of the level of (non-reactive; i.e. bound or masked) Aβ-specific IgM into the other direction have a marker function with respect to AD. With the present method, when the "healthy" level of reactive Aβ-specific IgG is set to 100%, a significant decrease in reactive Aβ-specific IgG, e.g. a decrease to 70% and lower, to 50% and lower or to 30% and lower, is indicative of development of AD. On the other hand, when the "healthy" level of Aβ-specific IgM is set to 100%, an increase in total IgM (reactive + unreactive) of at least 30%, e.g. at least 50% or at least 100%, in a blood sample indicates development of AD.

Accordingly, a preferred field of use of the present invention is the diagnosis of Alzheimer's disease (AD). However, the present invention is usable for all pathologies connected to or being related to Aβ ("Aβ pathologies"), especially to pathologies where Aβ deposits are occurring in the course of the disease. Examples for such Aβ pathologies are Parkinson's dementia (PDD), Dementia with Lewy Bodies (DLB), Cerebral Amyloid Angiopathy (CAA), Inclusion body myositis (IBM; especially sporadic IBM (sIBM)), or chronic head trauma (e.g., boxing).

Since the present invention provides a marker for AD and even for the development of AD, it is possible to use this method for observing the development of the disease and the performance of possible treatment methods, especially whether the method of treatment enables to establish "healthy" or "healthier" levels of Aβ-specific antibodies, especially IgG or IgM.

The present method is therefore preferably used for the monitoring of AD patients, especially AD patients who are treated with medicaments for curing or ameliorating AD. The present method can be successfully applied for observing patients in clinical trials for AD vaccines (e.g. with AD mimotopes according to WO 2004/062556 A, WO2006/005707 A, WO2009/149486 A and WO 2009/149485 A; or with Aβ-derived vaccines according to WO 99/27944 A) or Aβ-targeting disease-modifying drugs.

The method according to the present invention can also be used for evaluating the risk of developing AD or for detecting early stage AD. With the present invention, it is in principle made possible to detect changes in the immunological set-up of patients with respect to Aβ-specific autoantibodies at a significantly earlier point in time than cognitive impairments. This could allow a significant improvement of early diagnosis of AD with respect to a much larger part of the population if established in routine testing format. This makes patients eligible for early stage treatment regimens and/or prevention (or delay) strategies for AD, especially vaccinations.

According to another aspect, the present invention relates to a kit for performing the method according to the present invention comprising
- Aβ-variant aggregates, and
- a sample container, especially for human samples (e.g. blood, serum, plasma).

Preferably, the kit according to the present invention may further contain means for detecting Aβ-variant aggregates being bound to Aβ-specific antibodies, preferably secondary antibodies, especially labelled secondary antibodies, e.g. anti-IgG- or anti-IgM-antibodies). Further components can be standard samples, positive and/or negative controls, instructions for use and suitable packaging means (e.g. stable boxes, coloured vials, etc.).

The present invention is further illustrated by the following examples and the drawing figures, yet without being restricted thereto.

### EXAMPLES:

### Materials and Methods

### Detection of Aβ-specific antibodies using ELISA

Different Aβ-variant peptides (Group 1 peptide: DA-Aβ25-35-AG; Group 2 peptide Aβ16-20-carboxymethyl-Cys-AA-Aβ24-28-AA-Aβ31-36; Group 3 peptides Aβ16-36 and HKQ-Aβ16-36-GI; Group 4 peptides Aβ11-36 and DYKDDDDKGY-Aβ11-36) purchased from EMC) are diluted in 100 mM NaHCO₃ (pH 9.2) at a concentration of 5µg/ml and coated on Maxisorp 96-well plates overnight. To prevent unspecific binding plates are blocked using 1% BSA/PBS at 37°C for 1h. The ELISA is performed with a serial dilution of human sera samples (starting with a dilution of 1:10) or with adding IVIG in concentrations ranging from 1 mg/ml down to 10 µg/ml diluted in binding buffer (PBS/0.1% BSA/0.1% Tween20) at 37°C for 1h. After repeated washing steps (3x) with PBS/0.1% Tween20 the secondary anti-human Ig HRP (0.5µg/ml) detection-antibody is added for 1h at 37°C. Samples are washed again 3x and ABTS (0.68 mM in 0.1 M citric acid pH 4.3) is added for 30 min for assay development before OD-measurement on plate reader (Biotek - Gen5 Program) at wave length 405 nm.

The amino acid sequences of these Aβ-variants according to the present invention are therefore as follows:
Group 1 peptide (SEQ ID No.6): 
Asp Ala Gly Ser Asn Lys Gly Ala Ile Ile Gly Leu Met Ala Gly
1               5                   10                  15
Group 2 peptide (SEQ ID No.7; Xaa = carboxymethyl-Cys): 
Lys Leu Val Phe Xaa Val Gly Ser Asn Lys Ile Ile Gly Leu Met Val
1               5                   10                  15
Group 3 peptide (SEQ ID No.8): 
Lys Leu Val Phe Phe Ala Glu Asp Val Gly Ser Asn Lys Gly Ala Ile
1               5                   10                  15
Ile Gly Leu Met Val
            20
Group 3 peptide (SEQ ID No.9): 
His Lys Gln Lys Leu Val Phe Phe Ala Glu Asp Val Gly Ser Asn Lys
1               5                   10                  15
Gly Ala Ile Ile Gly Leu Met Val Gly Ile
            20                  25
Group 4 peptide (SEQ ID No.10): 
Glu Val His His Gln Lys Leu Val Phe Phe Ala Glu Asp Val Gly Ser
1               5                   10                  15
Asn Lys Gly Ala Ile Ile Gly Leu Met Val
            20                  25
Group 4 peptide (SEQ ID No.11):

Asp Tyr Lys Asp Asp Asp Asp Lys Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val
        35

### Detection of Aβ-specific antibodies using FACS analysis

Prior to analysis, the lyophilized β-amyloid peptides are subjected to a disaggregation procedure. For this purpose lyophilized Aβ species are dissolved in 1% NH₄OH (pH 11). Dissolved Aβ peptides are subsequently aliquoted and stored at -20°C. To induce the formation of aggregates, the different Aβ-species are incubated at a concentration of 20 µM in an aqueous solution (pH 5) at 37°C on shaker (350 rpm) overnight in an Eppendorf tube. The formation of Aβ-variant aggregates could be confirmed by Thioflavin T (ThT) staining in FACS (FSC/FL1). Aggregated Aβ-species are diluted to 0.15 µM in sterile filtered 0.5% BSA and pre-incubated for 30 min in a final volume of 95 µl on a 96-well plate. 5 µl of pre-diluted human plasma or Abs (in 0.5% BSA/PBS) is added to 95 µl of Aβ-variant aggregate-solution. The final dilution of plasma ranged from 1:1000 up to 1:10.000. For monoclonal antibodies, concentrations below 0.5 µg/ml are used. After 45 or 60min incubation at room temperature (RT) on shaker (350 rpm) 200 µl of 0.5% BSA/PBS is added in every well and centrifuged for 5 min at 3000 rpm (96-well plate-centrifuge). Supernatant is removed and washing step is repeated again with 200 µl of 0.5% BSA/PBS. After the second wash the SN is discarded and pellet is re-suspended in 100µl 0.5% BSA/PBS containing a 1:1000 dilution of labelled secondary anti-IgG (H+L)-PE or a 1:500 dilution of anti-IgM, Fc5µ antibody (both Jackson Immuno Research). Samples are incubated for another 45 or 60 min at RT on shaker (350 rpm) and measured on FACS Canto equipped with high-throughput sampler (HTS). Aggregates are gated in FSC/SSC and mean respectively median fluorescence intensity (MFI) is measured and evaluated in FL2-PE channel, and reactivity of ThT to Aβ-variant aggregates is measures and evaluated in FL1-FITC channel, using FACS Diva software.

### Demasking

To disrupt the binding of Aβ specific auto-antibodies to Aβ likely present in patient sera and, therefore, preventing detection of these Aβ bound auto-antibodies by antigen-based methods (like ELISA or FACS), sera are pre-diluted in 10mM Glycin pH2.6 at a dilution of 1:16.7 for 5min.

To disrupt the potential binding of Aβ antigens to auto-antibodies sera are pre-diluted in 10mM Glycin pH2.6 at a dilution of 1:16.7 for 5min. 5µl of the acidified serum are then co-incubated with 3µl of Aβ1-42 (100µg/ml) for another 5min. Then the mixture is neutralized by addition of 92µl of 0.5%BSA/PBS and incubated for 20 to 60 min. Washing steps and incubation with secondary antibody are performed as described above for non-demasked serum.

### Results

### Aβ-variant aggregates: Oligomerization and fibril formation

The formation of Aβ-variant aggregates (including Aβ oligomers, fibrils and fibrillar aggregates) from monomeric Aβ has been intensively investigated under multiple conditions in the recent years. It has been found that aggregation of Aβ peptides is very much dependent on different conditions including pH, temperature, buffer composition and protein concentration. Aggregation starts with the formation of β-hairpins from monomers leading to soluble oligomers. Conformational transition into parallel β-sheets then leads to the formation of fibrils and fibrillar aggregates, which can be precipitated by centrifugation. Recent findings have shown that Aβ aggregates produced at pH 7.4 comprise fibrils with a 5 nm wide filament with a slight tendency to laterally associate into bundles (into 15 to 25 nm wide filaments). The length of such a single fibril lies in the range of sub-micrometer (50-100 nm) up to 10-15µm as determined by transmission electron microscopy. One characteristic of these Aβ fibrils is that they specifically intercalate with a fluorescent dye, the Thioflavin T (ThT).

According to the present invention, Aβ-variant aggregates of four different Aβ-peptide variants, including (Group 1 peptide: DA-Aβ25-35-AG; Group 2 peptide Aβ16-20-carboxymethyl-Cys-AA-Aβ24-28-AA-Aβ31-36; Group 3 peptides Aβ16-36 and HKQ-Aβ16-36-GI; Group 4 peptides Aβ11-36 and DYKDDDDKGY-Aβ11-36), which intercalate with ThT, are generated. These Aβ-variant aggregates can be detected using FACS-analysis. As described in MM for this purpose seedless soluble Aβ-variant peptides are incubated for 20 h at 37°C at a concentration of 20 µM. The FACS assay developed according to the present invention allows the analysis of the in vitro generated Aβ-variant aggregates. The size distribution of Aβ-variant aggregates (defined by forward scatter FSC-A) is analyzed using calibrated size beads ranging from 1 to 15 µm (Flow Cytometry Size Calibration Kit (Cat.# F-13838) by Molecular probes) (Figure 1 lower panel). Using this analysis it is shown that the size of generated Aβ-variant aggregates ranged as expected from sub-micrometer range up to 10 µm in which most of the generated aggregates range from ∼500 nm up to 3 µm.

### Reactivity of mAbs with Aβ-variant aggregates

To define whether Aβ-variant aggregates allow the binding of Aβ-specific antibodies and to determine whether such an interaction can be monitored using the here described FACS-based assay another set of experiments is undertaken. For this purpose, Aβ-variant aggregates according to the present invention (SEQ ID NOs. 8 to 11) are generated and are incubated with monoclonal antibodies specific either for Aβ1-42 (3A5) or specific for all Aβ-variant aggregates containing the sequence Aβ17-21 (4G8). As can be shown in FACS histograms, the described FACS-based assay allows the detection of Aβ specific antibodies in a specific manner.

### Defining the sensitivity of different detection methods for Aβ binding of human auto-antibodies using IVIG

IVIG (intravenous immunoglobulin) is a commercially available blood product. It contains the pooled IgG fraction extracted from plasma derived from healthy donors (human plasma from at least 1000 donors). It has been shown that IVIG preparations contain naturally occurring antibodies (auto-antibodies) specific for Aβ-peptides.

The aim of this experiment is to define and compare the detection limits of two independent detection methods (ELISA and the FACS-based assay) for Aβ-reactivity of IVIG (IVIG-Subcuvia, purchased from Baxter, Austria). Aβ variants (SEQ ID NOs. 8 to 11) are therefore immobilized onto Maxisorp microtiter plates for ELISA measurements. Alternatively, Aβ aggregates are generated for FACS analysis. Subsequently, different IVIG dilutions are applied to the individual systems and OD values in case of ELISA or fluorescence intensity (MFI values) in case of FACS assay are defined. For comparison reasons signal to noise ratio is evaluated for all IVIG concentrations and signals are expressed as fold-background-signal (xBG). Using ELISA as detection method 1000 µg/ml IVIG gives a clear signal above background whereas the signal induced by 100 µg/ml IVIG is only 2 times background at maximum. 10 µg/ml IVIG does not result in a signal greater than background (Figure 3B). The FACS-based assay provides signals that are much higher than signals delivered by ELISA methods. This shows that the newly developed FACS-based assay to detect Aβ-specific auto-antibodies is at least 100 times more sensitive than conventional assays such as ELISA.

### Defining anti-beta amyloid antibodies in human blood derived from healthy donors and AD patients

### a. Reactivity of IgG to Aβ-variant aggregates

Serum samples derived either from healthy individuals (n=13, ∼30 to 50 years old) or AD patients (n=48, ∼70 years of age) are analysed for naturally occurring Aβ-specific antibody content using Aβ-variant aggregates and FACS analysis as described above. Naturally occurring antibodies specific for Aβ-variant aggregates are detected in all samples tested. However, the concentration of such antibodies is significantly lower in blood samples derived from AD patients when compared to serum samples derived from healthy subjects.

### b. Reactivity of IgM to Aβ-variant aggregates

In a next set of experiments Aβ-variant aggregate specific IgM reactivity is defined in serum samples derived either from healthy individuals or AD patients, using the same sera as described above. Naturally occurring antibodies of IgM isotype specific for Aβ-variant aggregates (SEQ ID NOs. 8 to 11) are detected in all samples tested. But in contrast to IgG reactivity, serum samples derived from healthy controls do not have higher IgM reactivity to Aβ-variant aggregates than serum samples derived from AD patients.

### d. Reactivity of IgM to Aβ-variant aggregates after demasking

In previous studies it has been shown that Aβ-specific auto-antibodies, mainly of the IgM isotype, can be occupied with the Aβ antigen to build an immune complex that is stable and is circulating in the human blood (WO 2010/128139 A1; Marcello et al., 2009; Lindhagen-Persson et al., PLoS ONE 5 (2010): e13928). To define whether Aβ antigens potentially bind to the auto-antibodies may block the reactivity of these antibodies to the in vitro generated Aβ-variant aggregates, the individual sera are subjected to a demasking procedure as described in material and methods. Using low pH potential immune complexes are disrupted resulting in removal of Aβ antigens from antibody binding domains (antigen dissociation). Thus a demasking procedure could result in higher auto-antibody signals in the FACS based assay if immune complexes are present in serum samples. Interestingly, IgM reactivity of AD patient sera increases significantly after demasking whereas reactivity of healthy control sera does not change compared to untreated sera. This is in contrast to that what is measured in case of IgG reactivity to Aβ-variant aggregates. Sera derived from healthy individuals show higher IgG reactivity to Aβ-variant aggregates than sera derived from AD patients (in this case sera are treated with low pH).

### Conclusions

It can be stated that the higher sensitivity of the FACS-aggregation-assay according to the present invention is directly connected to the different aggregation status of the Aβ-variants according to the present invention. For BIAcore analysis a freshly dissolved and biotinylated Aβvariant is used for immobilization on a streptavidin chip. This method favours the binding of monomeric Aβ-variants on the chip surface because the peptide is immobilized immediately without preincubation and also the Biotin-tag decelerates the formation of aggregates. Also the coating of Aβ-variants at pH9 on Maxisorp plates seems to favour the monomeric form of the variant although the coating of some aggregates cannot be excluded. In these assays the affinities between antibodies and Aβ-variants are responsible for the limit of detection.

In contrast to these two methods for the FACS-based assay according to the present invention Aβ-variants aggregates are specifically induced and used for detection of antibodies specific for Aβ present in IVIG. These bigger molecules offer multiple antibody binding sites. The resulting avidity effect (the sum of multiple synergistic low affine antibody-antigen interactions) can account for the higher sensitivity of this assay and by leading to the detection of low-affine antibodies within the IVIG fraction. Reactivity of IVIG to Aβ-variant aggregates can also be explained by the existence of an epitope solely present on the aggregated form of Aβ.

The examples clearly show the superiority of the present method over the methods currently available in the field, especially with respect to analytical properties, such as specificity and selectivity.

## Claims

1. A method for detecting Aβ-specific antibodies in a biological sample comprising the following steps:
- contacting the sample with Aβ-variant aggregates or with particles having Aβ-variant aggregate like surfaces and allowing the Aβ-specific antibodies to bind to the Aβ-variant aggregates, and
- detecting the Aβ-specific antibodies bound to the Aβ-variant aggregates by a single particle detection technique, preferably by fluorescence activated cell sorting (FACS),
wherein the Aβ-variant is selected from the group consisting of
- a mutant of the Aβ-1-40- or the Aβ-1-42-peptide containing at least one mutation at an amino acid position other than amino acids 16 to 20, 25 to 28 and 31 to 35,
- a naturally occurring Aβ-peptide other than Aβ-1-40, Aβ-1-42, Aβ-1-43, Aβ-3-42 or Aβ-p(E)3-42,
- a truncated form of the Aβ-1-40- or the Aβ-1-42-peptide, wherein the truncation is at amino acid positions other than amino acids 16 to 35 and wherein the truncation is at least one amino acid and at most 20 amino acids; or
- combinations thereof, especially combinations of at least one mutation and at least one truncation of the Aβ-1-40- or the Aβ-1-42-peptide.

2. Method according to claim 1 **characterised in that** the Aβ-specific antibodies are human antibodies, preferably human IgG or IgM antibodies, especially human IgG antibodies.

3. Method according to claim 1 or 2, **characterised in that** the Aβ-specific antibodies are autoantibodies.

4. Method according to any one of claims 1 to 3, **characterised in that** the Aβ-variant aggregates have a size of 50 nm to 15 µm, preferably from 100 nm to 10 µm, especially from 200 nm to 5 µm.

5. Method according to any one of claims 1 to 4, **characterised in that** the Aβ-variant aggregates have been prepared by incubating Aβ-variant peptides at a pH of 2 to 9 for at least 20 min, preferably at least 1 h, especially at least 4 h.

6. Method according to any one of claims 1 to 5, **characterised in that** the Aβ-variant aggregates are present for contacting the sample with Aβ-variant aggregates in an amount of 0.001 to 1 µM, preferably 0.01 to 0.1 µM.

7. Method according to any one of claims 1 to 6, **characterised in that** the biological sample is human blood or a sample derived from human blood, preferably human serum or human plasma; human cerebrospinal fluid or human lymph.

8. Method according to any one of claims 1 to 7, **characterised in that** the Aβ-variant aggregates are contacted with the sample for at least 10 min, preferably from 10 min to 24 h, especially from 20 min to 2 h.

9. Method according to any one of claims 1 to 8, **characterised in that** a demasking step is performed on the Aβ-specific antibodies in the sample before contacting the sample with Aβ-variant aggregates, especially if IgM antibodies are detected.

10. Method according to any one of claims 1 to 9, **characterised in that** the particles having Aβ-variant aggregate like surfaces are beads with Aβ-variant aggregates immobilised on their surface, especially magnetic beads.

11. Use of a method according to any one of claims 1 to 10 for the diagnosis of Alzheimer's disease (AD).

12. Use of a method according to any one of claims 1 to 10 for the monitoring of AD patients, especially AD patients which are treated with medicaments for curing or ameliorating AD.

13. Use of a method according to any one of claims 1 to 10 for evaluating the risk of developing AD or for detecting early stage AD.

14. Use of a method according to any one of claims 1 to 10 for the diagnosis of Parkinson's dementia (PDD), Dementia with Lewy Bodies (DLB), Cerebral Amyloid Angiopathy (CAA), Inclusion body myositis (IBM), or chronic head trauma.

15. Kit for performing the method according to any one of claims 1 to 10 comprising
- Aβ-variant aggregates, and
- a sample container.


SEQUENCE LISTING

<110>  Affiris AG

<120>  Method for detecting A-beta-specific antibodies in a biological
       sample

<130>  R 63823

<160>  11

<170>  PatentIn version 3.5

<210>  1
<211>  42
<212>  PRT
<213>  artificial

<220>
<223>  artificially produced polypeptide derived from A-beta

<400>  1

<210>  2
<211>  40
<212>  PRT
<213>  artificial

<220>
<223>  artificially produced polypeptide derived from A-beta

<400>  2

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  3
<211>  43
<212>  PRT
<213>  artificial

<220>
<223>  artificially produced polypeptide derived from A-beta

<400>  3

Asp Ala Glu Phe Arg His Asp Ser Gly Tyr Glu Val His His Gln Lys

1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala Thr
        35                  40

<210>  4
<211>  40
<212>  PRT
<213>  artificial

<220>
<223>  artificially produced polypeptide derived from A-beta

<400>  4

Glu Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val Ile Ala
        35                  40

<210>  5
<211>  40
<212>  PRT
<213>  artificial

<220>
<223>  artificially produced polypeptide derived from A-beta

<220>
<221>  VARIANT
<222>  (1)..(1)
<223>  pyro-glutamate

<400>  5

Xaa Phe Arg His Asp Ser Gly Tyr Glu Val His His Gln Lys Leu Val
1               5                   10                  15

Phe Phe Ala Glu Asp Val Gly Ser Asn Lys Gly Ala Ile Ile Gly Leu
            20                  25                  30

Met Val Gly Gly Val Val Ile Ala
        35                  40

<210>  6
<211>  15
<212>  PRT
<213>  artificial

<220>
<223>  artificially produced polypeptide derived from A-beta

<400>  6

Asp Ala Gly Ser Asn Lys Gly Ala Ile Ile Gly Leu Met Ala Gly
1               5                   10                  15


<210>  7
<211>  16
<212>  PRT
<213>  artificial

<220>
<223>  artificially produced polypeptide derived from A-beta


<220>
<221>  VARIANT
<222>  (5)..(5)
<223>  carboxymethyl-Cys

<400>  7

Lys Leu Val Phe Xaa Val Gly Ser Asn Lys Ile Ile Gly Leu Met Val
1               5                   10                  15


<210>  8
<211>  21
<212>  PRT
<213>  artificial

<220>
<223>  artificially produced polypeptide derived from A-beta

<400>  8

Lys Leu Val Phe Phe Ala Glu Asp Val Gly Ser Asn Lys Gly Ala Ile
1               5                   10                  15


Ile Gly Leu Met Val
            20


<210>  9
<211>  26
<212>  PRT
<213>  artificial

<220>
<223>  artificially produced polypeptide derived from A-beta

<400>  9

His Lys Gln Lys Leu Val Phe Phe Ala Glu Asp Val Gly Ser Asn Lys
1               5                   10                  15


Gly Ala Ile Ile Gly Leu Met Val Gly Ile
            20                  25


<210>  10
<211>  26
<212>  PRT
<213>  artificial

<220>
<223>  artificially produced polypeptide derived from A-beta

<400>  10

Glu Val His His Gln Lys Leu Val Phe Phe Ala Glu Asp Val Gly Ser
1               5                   10                  15

Asn Lys Gly Ala Ile Ile Gly Leu Met Val
            20                  25

<210>  11
<211>  36
<212>  PRT
<213>  artificial

<220>
<223>  artificially produced polypeptide

<400>  11

Asp Tyr Lys Asp Asp Asp Asp Lys Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val
        35

SEQUENCE LISTING
<110> Affiris AG
<120> Method for detecting A-beta-specific antibodies in a biological
sample
<130> R 63823
<160> 11
<170> PatentIn version 3.5
<210> 1
<211> 42
<212> PRT
<213> Artificial Sequence
<220>
<223> artificially produced polypeptide derived from A-beta
<400> 1
<210> 2
<211> 40
<212> PRT
<213> Artificial Sequence
<220>
<223> artificially produced polypeptide derived from A-beta
<400> 2
Asp Ala Glu Phe Arg His Asp Ser Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val Gly Gly Val Val
        35                  40
<210> 3
<211> 43
<212> PRT
<213> Artificial Sequence
<220>
<223> artificially produced polypeptide derived from A-beta
<400> 3
Asp Ala Glu Phe Arg His Asp Ser Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val Gly Gly Val Val Ile Ala Thr
        35                  40
<210> 4
<211> 40
<212> PRT
<213> Artificial Sequence
<220>
<223> artificially produced polypeptide derived from A-beta
<400> 4
Glu Phe Arg His Asp Ser Gly Tyr Glu Val His His Gln Lys Leu Val
1               5                   10                  15
Phe Phe Ala Glu Asp Val Gly Ser Asn Lys Gly Ala Ile Ile Gly Leu
            20                  25                  30
Met Val Gly Gly Val Val Ile Ala
        35                  40
<210> 5
<211> 40
<212> PRT
<213> Artificial Sequence

<220>
<223> artificially produced polypeptide derived from A-beta
<220>
<221> VARIANT
<222> (1)..(1)
<223> pyro-glutamate
<400> 5
Xaa Phe Arg His Asp Ser Gly Tyr Glu Val His His Gln Lys Leu Val
1               5                   10                  15
Phe Phe Ala Glu Asp Val Gly Ser Asn Lys Gly Ala Ile Ile Gly Leu
            20                  25                  30
Met Val Gly Gly Val Val Ile Ala
        35                  40
<210> 6
<211> 15
<212> PRT
<213> Artificial Sequence
<220>
<223> artificially produced polypeptide derived from A-beta
<400> 6
Asp Ala Gly Ser Asn Lys Gly Ala Ile Ile Gly Leu Met Ala Gly
1               5                   10                  15
<210> 7
<211> 16
<212> PRT
<213> Artificial Sequence
<220>
<223> artificially produced polypeptide derived from A-beta
<220>
<221> VARIANT
<222> (5)..(5)
<223> carboxymethyl-Cys
<400> 7
Lys Leu Val Phe Xaa Val Gly Ser Asn Lys Ile Ile Gly Leu Met Val
1               5                   10                  15
<210> 8
<211> 21
<212> PRT
<213> Artificial Sequence
<220>
<223> artificially produced polypeptide derived from A-beta
<400> 8
Lys Leu Val Phe Phe Ala Glu Asp Val Gly Ser Asn Lys Gly Ala Ile
1               5                   10                  15
Ile Gly Leu Met Val
            20
<210> 9
<211> 26
<212> PRT
<213> Artificial Sequence
<220>
<223> artificially produced polypeptide derived from A-beta
<400> 9
His Lys Gln Lys Leu Val Phe Phe Ala Glu Asp Val Gly Ser Asn Lys
1               5                   10                  15
Gly Ala Ile Ile Gly Leu Met Val Gly Ile
            20                  25
<210> 10
<211> 26
<212> PRT
<213> Artificial Sequence
<220>
<223> artificially produced polypeptide derived from A-beta

<400> 10
Glu Val His His Gln Lys Leu Val Phe Phe Ala Glu Asp Val Gly Ser
1               5                   10                  15
Asn Lys Gly Ala Ile Ile Gly Leu Met Val
            20                  25
<210> 11
<211> 36
<212> PRT
<213>  Artificial Sequence
<220>
<223> artificially produced polypeptide
<400> 11
Asp Tyr Lys Asp Asp Asp Asp Lys Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val
        35